# EUROPEAN PATENT SPECIFICATION

(11) **EP 1 676 922 B1**
(45) Date of publication and mention of the grant of the patent: **17.09.2008**
(21) Application number: 05022193.6
(22) Date of filing: 04.11.1998
(51) Int. Cl.: C12N 15/82, C07K 14/325, C12N 15/32, C12N 15/62

(54) **Plant-optimized genes encoding pesticidal toxins**
Pflanzen-optimierte Gene, welche für Toxine mit pestizider Wirkung kodieren
Gènes à expression optimisée dans les végétaux codant pour des toxines pesticides

(30) Priority: 12.11.1997 US 65215 P; 02.03.1998 US 76445 P; 23.10.1998 US 178252
(43) Date of publication of application: 05.07.2006
(62) Divisional of application: 98956551.0
(73) Proprietor: Mycogen Corporation, Indianapolis, IN 46268 (US)
(72) Inventor: Cardineau, Guy A., Poway, CA 92064 (US); Stelman, Steven J., San Diego, CA 92126 (US); Narva, Kenneth E., San Diego, CA 92130 (US)
(74) Representative: Perry, Robert Edward

(56) References cited:
- WO-A-93/07278
- WO-A-95/30752
- WO-A-95/30753
- US-A- 5 508 264
- US-A- 5 530 195

## Description

### Background of the Invention

Insects and other pests cost farmers billions of dollars annually in crop losses and in the expense of keeping these pests under control. The losses caused by insect pests in agricultural production environments include decrease in crop yield, reduced crop quality, and increased harvesting costs.

Chemical pesticides have provided an effective method of pest control; however, the public has become concerned about the amount of residual chemicals which might be found in food, ground water, and the environment. Therefore, synthetic chemical pesticides are being increasingly scrutinized, and correctly so, for their potential toxic environmental consequences. Synthetic chemical pesticides can poison the soil and underlying aquifers, pollute surface waters as a result of runoff, and destroy non-target life forms. Synthetic chemical control agents have the further disadvantage of presenting public safety hazards when they are applied in areas where pets, farm animals, or children may come into contact with them. They may also provide health hazards to applicants, especially if the proper application techniques are not followed. Regulatory agencies around the world are restricting and/or banning the uses of many pesticides and particularly the synthetic chemical pesticides which are persistent in the environment and enter the food chain. Examples of widely used synthetic chemical pesticides include the organochlorines, *e*.*g.*, DDT, mirex, kepone, lindane, aldrin, chlordane, aldicarb, and dieldrin; the organophosphates, *e*.*g*., chlorpyrifos, parathion, malathion, and diazinon; and carbamates. Stringent new restrictions on the use of pesticides and the elimination of some effective pesticides from the market place could limit economical and effective options for controlling costly pests.

Because of the problems associated with the use of synthetic chemical pesticides, there exists a clear need to limit the use of these agents and a need to identify alternative control agents. The replacement of synthetic chemical pesticides, or combination of these agents with biological pesticides, could reduce the levels of toxic chemicals in the environment.

A biological pesticidal agent that is enjoying increasing popularity is the soil microbe *Bacillus thuringiensis* (*B*.*t*). The soil microbe *Bacillus thuringiensis* (*B*.*t*.) is a Gram-positive, spore-forming bacterium. Most strains of *B.t.* do not exhibit pesticidal activity. Some *B.t.* strains produce, and can be characterized by, parasporal crystalline protein inclusions. These "δ-endotoxins," which typically have specific pesticidal activity, are different from exotoxins, which have a non-specific host range. These inclusions often appear microscopically as distinctively shaped crystals. The proteins can be highly toxic to pests and are specific in their toxic activity.

Preparations of the spores and crystals of *B*. *thuringiensis* subsp. *kurstaki* have been used for many years as commercial insecticides for lepidopteran pests. For example, *B*. *thuringiensis* var. *kurstaki* HD-1 produces a crystalline δ-endotoxin which is toxic to the larvae of a number of lepidopteran insects.

The cloning and expression of a *B.t.* crystal protein gene in *Escherichia coli* was described in the published literature more than 15 years ago (Schnepf, H.E., H.R. Whiteley [1981] Proc. Natl. Acad. Sci. USA 78:2893-2897.). U.S. Patent No. 4,448,885 and U.S. Patent No. 4,467,036 both disclose the expression of *B*.*t*. crystal protein in *E*. *coli*. Recombinant DNA-based *B*.*t*. products have been produced and approved for use.

Commercial use of *B.t.* pesticides was originally restricted to a narrow range of lepidopteran (caterpillar) pests. More recently, however, investigators have discovered *B.t.* pesticides with specificities for a much broader range of pests. For example, other species of B.t., namely *israelensis* and *morrisoni* (a.k.a. *tenebrionis*, a.k.a. *B.t.* M-7), have been used commercially to control insects of the orders Diptera and Coleoptera, respectively (Gaertner, F.H. [1989] "Cellular Delivery Systems for Insecticidal Proteins: Living and Non-Living Microorganisms," in Controlled Delivery of Crop Protection Agents, R.M. Wilkins, ed., Taylor and Francis, New York and London, 1990, pp. 245-255).

New subspecies of *B*.*t*. have now been identified, and genes responsible for active δ-endotoxin proteins have been isolated and sequenced (Höfte, H., H.R Whiteley [1989] Microbiological Reviews 52(2):242-255). Höfte and Whiteley classified *B.t.* crystal protein genes into four major classes. The classes were *cry*I (Lepidoptera-specific), *cry*II (Lepidoptera-and Diptera-specific), *cry*III (Coleoptera-specific), and *cry*IV (Diptera-specific). The discovery of strains specifically toxic to other pests has been reported (Feitelson, J.S., J. Payne, L. Kim [1992] Bio/Technology 10:271-275). For example, the designations CryV and CryVI have been proposed for two new groups of nematode-active toxins.

Many *Bacillus thuringiensis* δ-endotoxin crystal protein molecules are composed of two functional segments. For these proteins, the protease-resistant core toxin is the first segment and corresponds to about the first half of the protein molecule. The three-dimensional structure of a core segment of a CryIIIA *B*.*t*. δ-endotoxin is known, and it was proposed that all related toxins have that same overall structure (Li, J., J. Carroll, D.J. Ellar [1991] Nature 353:815-821). The second half of the molecule is often referred to as the "protoxin segment." The protoxin segment is believed to participate in toxin crystal formation (Arvidson, H., P.E. Dunn, S. Strand, A.I. Aronson [1989] Molecular Microbiology 3:1533-1534; Choma, C.T., W.K. Surewicz, P.R. Carey, M. Pozsgay, T. Raynor, H. Kaplan [1990] Eur. J. Biochem. 189:523-527). The full 130 kDa toxin molecule is typically processed to the resistant core segment by proteases in the insect gut. The protoxin segment may thus convey a partial insect specificity for the toxin by limiting the accessibility of the core to the insect by reducing the protease processing of the toxin molecule (Haider, M.Z., B.H. Knowles, DJ. Ellar [1986] Eur. J. Biochem. 156:531-540) or by reducing toxin solubility (Aronson, A.I., E.S. Han, W. McGaughey, D. Johnson [1991] Appl. Environ. Microbiol. 57:981-986).

The 1989 nomenclature and classification scheme of Höfte and Whiteley was based on both the deduced amino acid sequence and the host range of the toxin. That system was adapted to cover 14 different types of toxin genes which were divided into five major classes. The number of sequenced *Bacillus thuringiensis* crystal protein genes currently stands at more than 50. A revised nomenclature scheme has been proposed which is based solely on amino acid identity (Crickmore et al. [1996] Society for Invertebrate Pathology, 29th Annual Meeting, IIIrd International Colloquium on Bacillus thuringiensis, University of Cordoba, Cordoba, Spain, September 1-6,1996, abstract). The mnemonic "cry" has been retained for all of the toxin genes except cytA and cytB, which remain a separate class. Roman numerals have been exchanged for Arabic numerals in the primary rank, and the parentheses in the tertiary rank have been removed. Many of the original names have been retained, although a number have been reclassified.

With the use of genetic engineering techniques, new approaches for delivering *B*.*t*. toxins to agricultural environments are under development, including the use of plants genetically engineered with *B*.*t*. toxin genes for insect resistance and the use of stabilized, microbial cells as delivery vehicles of *B*.*t*. toxins (Gaertner, F.H., L. Kim [1988] TIBTECH 6:S4-S7). Thus, isolated *B*.*t*. endotoxin genes are becoming commercially valuable.

Various improvements have been achieved by modifying *B*.*t*. toxins and/or their genes. For example, U.S. Patent Nos. 5,380,831 and 5,567,862 relate to the production of synthetic insecticidal crystal protein genes having improved expression in plants.

Obstacles to the successful agricultural use of *B*.*t*. toxins include the development of resistance to *B*.*t*. toxins by insects. In addition, certain insects can be refractory to the effects of *B*.*t*. Such insects include the boll weevil and black cutworm, as well as adult insects of most species which previously have demonstrated no apparent significant sensitivity to *B*.*t*. δ-endotoxins.

Thus, resistance management strategies in *B*.*t*. plant technology have become of great interest. The discovery of *B*.*t*. isolates and new uses of known *B*.*t*. isolates remains an empirical, unpredictable art. There remains a great need for new toxin genes that can be successfully expressed at adequate levels in plants in a manner that will result in the effective control of insects and other pests.

### Summary of the Invention

A polynucleotide according to the present invention comprises the nucleotide sequence SEQ ID NO. 1. This is a polynucleotide sequence for a gene designed 1AC1AB-B-PO, which is optimized for expression in plants. This gene encodes a chimeric CrylAc (N-terminal)/Cry1Ab (protoxin) toxin.

The polynucleotide sequence of the present invention has certain modifications, compared to wild-type sequences, that make it particularly well-suited for optimized expression in plants. Using techniques known to those skilled in the art, the polynucleotide sequences described herein can be used to transform plants in order to confer pest resistance upon the plants.

### Description of the Invention

It should be apparent to a person skilled in this art that, given the sequence set forth herein, a gene of the subject invention can be obtained through several means. In a preferred embodiment, the subject gene may be constructed synthetically by using a gene synthesizer. The specific gene exemplified herein can also be obtained by modifying, according to the teachings of the subject invention, certain wild-type genes (for example, by point-mutation techniques) from certain isolates deposited at a culture depository as discussed below.

The polynucleotides of the subject invention can be used to form complete "genes" to encode proteins or peptides in a desired host cell. For example, as the skilled artisan would readily recognize, the polynucleotides of the subject invention are shown without stop codons. Also, the subject polynucleotides can be appropriately placed under the control of a promoter in a host of interest, as is readily known in the art.

As the skilled artisan would readily recognize, DNA can exist in a double-stranded form. In this arrangement, one strand is complementary to the other strand and vice versa. The "coding strand" is often used in the art to refer to the strand having a series of codons (a codon is three nucleotides that can be read three-at-a-time to yield a particular amino acid) that can be read as an open reading frame (ORF) to form a protein or peptide of interest. In order to express a protein *in vivo*, a strand of DNA is typically translated into a complementary strand of RNA which is used as the template for the protein. As DNA is replicated in a plant (for example) additional, complementary strands of DNA are produced. Thus, the subject invention includes the use of either the exemplified polynucleotides shown in the attached sequence listing or the complementary strands. RNA and PNA (peptide nucleic acids) that are functionally equivalent to the exemplified DNA are included in the subject invention. that the subject invention includes not only the genes and sequences specifically exemplified herein but also equivalents and variants thereof (such as mutants, fusions, chimerics, truncations, fragments, and smaller genes) that exhibit the same or similar characteristics relating to expressing toxins in plants, as compared to those specifically disclosed herein. As used herein, "variants" and "equivalents" refer to sequences which have nucleotide (or amino acid) substitutions, deletions (internal and/or terminal), additions, or insertions which do not materially affect the expression of the subject genes, and the resultant pesticidal activity, in plants. Fragments retaining pesticidal activity are also included in this definition. Thus, polynucleotides that are smaller than those specifically exemplified are included in the subject invention, so long as the polynucleotide encodes a pesticidal toxin.

Genes can be modified, and variations of genes may be readily constructed, using standard techniques. For example, techniques for making point mutations are well known in the art. In addition, commercially available exonucleases or endonucleases can be used according to standard procedures, and enzymes such as *Bal*31 or site-directed mutagenesis can be used to systematically cut off nucleotides from the ends of these genes. Useful genes can also be obtained using a variety of restriction enzymes.

It should be noted that equivalent genes will encode toxins that have high amino acid identity or homology with the toxins encoded by the subject genes. The amino acid homology will be highest in critical regions of the toxin which account for biological activity or are involved in the determination of three-dimensional configuration which ultimately is responsible for the biological activity. In this regard, certain substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Table 1 provides a listing of examples of amino acids belonging to each class.

| Table 1. | |
|---|---|
| Class of Amino Acid | Examples of Amino Acids |
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the ability of plants to express the subject DNA sequences or from the biological activity of the toxin.

As used herein, reference to "isolated" polynucleotides refers to these molecules when they are not associated with the other molecules with which they would be found in nature and would include their use in plants. Thus, reference to "isolated and purified" signifies the involvement of the "hand of man" as described herein.

The toxin-encoding genes of the subject invention can be introduced into a wide variety of microbial or plant hosts. In some embodiments of the subject invention, transformed microbial hosts can be used in preliminary steps for preparing precursors, for example, that will eventually be used to transform, in preferred embodiments, plant cells and plants so that they express the toxins encoded by the genes of the subject invention. Microbes transformed and used in this manner are within the scope of the subject invention. Recombinant microbes may be, for example, *B*.*t*., *E*. *coli*, or *Pseudomonas*. Transformations can be made by those skilled in the art using standard techniques. Materials necessary for these transformations are disclosed herein or are otherwise readily available to the skilled artisan.

Thus, in preferred embodiments, expression of the toxin gene results, directly or indirectly, in the intracellular production and maintenance of the pesticide. When transformed plants are ingested by the pest, the pests will ingest the toxin. The result is a control of the pest.

The *B*.*t*. toxin gene can be introduced via a suitable vector into a host, preferably a plant host. There are many crops of interest, such as corn, wheat, rice, cotton, soybeans, and sunflowers. The genes of the subject invention are particularly well suited for providing stable maintenance and expression, in the transformed plant, of the gene expressing the polypeptide pesticide, and, desirably, provide for improved protection of the pesticide from environmental degradation and inactivation.

Obviously, a promoter region capable of expressing the gene in a plant is needed. Thus, for *in planta* expression, the DNA of the subject invention is under the control of an appropriate promoter region. Techniques for obtaining *in planta* expression by using such constructs is known in the art.

Genes encoding pesticidal toxins, as disclosed herein, can be inserted into plant cells using a variety of techniques which are well known in the art. For example, a large number of cloning vectors comprising a replication system in *E*. *coli* and a marker that permits selection of the transformed cells are available for preparation for the insertion of foreign genes into higher plants. The vectors comprise, for example, pBR322, pUC series, M13mp series, pACYC184, etc. Accordingly, the sequence encoding the *B*.*t*. toxin can be inserted into the vector at a suitable restriction site. The resulting plasmid is used for transformation into *E*. *coli*. The *E*. *coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids.

Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted. The use of T-DNA for the transformation of plant cells has been intensively researched and sufficiently described in EP 120 516; Hoekema (1985) In: The Binary Plant Vector System, Offset-durkkerij Kanters B.V., Alblasserdam, Chapter 5; Fraley et al., Crit. Rev. Plant Sci. 4:1-46; and An et al. (1985) EMBO J. 4:277-287.

Once the inserted DNA has been integrated in the genome, it is relatively stable there and, as a rule, does not come out again. It normally contains a selection marker that confers on the transformed plant cells resistance to a biocide or an antibiotic, such as kanamycin, G 418, bleomycin, hygromycin, or chloramphenicol, *inter alia*. The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation agent, fusion, injection, biolistics (microparticle bombardment), or electroporation as well as other possible methods. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the *vir* region necessary for the transfer of the T-DNA. Intermediate vectors cannot replicate themselves in Agrobacteria. The intermediate vector can be transferred into *Agrobacterium tumefaciens* by means of a helper plasmid (conjugation). Binary vectors can replicate themselves both in *E*. *coli* and in Agrobacteria. They comprise a selection marker gene and a linker or polylinker which are framed by the right and left T-DNA border regions. They can be transformed directly into Agrobacteria (Holsters et al. [1978] Mol. Gen. Genet. 163:181-187). The *Agrobacterium* used as host cell is to comprise a plasmid carrying a *vir* region. The *vir* region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

## Claims

1. A polynucleotide comprising SEQ ID NO:1.

2. A transformed host cell comprising a polynucleotide sequence as defined in claim 1.

3. A DNA construct comprising a promoter region capable of expression in a plant cell and, under the control of said promoter region, a polynucleotide sequence as defined in claim 1.

4. A method for controlling a pest, which comprises contacting said pest with a toxin produced by a transformed host, wherein said transformed host comprises a polynucleotide sequence as defined in claim 1.

## Patentansprüche

1. Polynucleotid umfassend SEQ ID NO:1.

2. Transformierte Wirtszelle umfassend eine Polynucleotidsequenz, wie in Anspruch 1 definiert.

3. DNA-Konstrukt umfassend eine Promotorregion, die zur Expression in einer Pflanze befähigt ist, und eine Polynucleotidsequenz, wie in Anspruch 1 definiert, unter der Kontrolle der Promotorregion.

4. Verfahren zur Schädlingsbekämpfung, welches umfasst das Kontaktieren des Schädlings mit einem Toxin, das von einem transformierten Wirt erzeugt wird, wobei der transformierte Wirt eine Polynucleotidsequenz, wie in Anspruch 1 definiert, umfasst.

## Revendications

1. Polynucléotide comprenant SEQ ID N° 1.

2. Cellule hôte transformée comprenant une séquence polynucléotidique comme définie dans la revendication 1.

3. Construction d'ADN comprenant une région promotrice capable d'expression dans une cellule végétale et, sous le contrôle de ladite région promotrice, une séquence polynucléotidique comme définie dans la revendication 1.

4. Procédé pour contrôler un nuisible qui comprend l'étape consistant à mettre en contact ledit nuisible avec une toxine produite par un hôte transformé, ledit hôte transformé comprenant une séquence polynucléotidique comme définie dans la revendication 1.


### SEQUENCE LISTING

<210> 1
   <211> 3468
   <212> DNA
   <213> Artificial Sequence
<220>
   <223> Synthetic B.t. toxin gene
<400> 1
   atggacaaca atcccaacat caacgagtgc attccttaca actgcctgag caaccctgag 60
   gttgaggtgc tgggtggaga acggattgag actggttaca cacctatcga catctcgttg 120
   tcacttaccc aattcctttt gtcagagttc gtgcccggtg ctggattcgt gcttggactt 180
   gtcgatatca tttggggaat ctttggtccc tctcaatggg acgcctttct tgtacagata 240
   gagcagttaa ttaaccaaag aatagaagaa ttcgctagga accaagccat ctcaaggtta 300
   gaaggcctca gcaaccttta ccagatttac gcagaatctt ttcgagagtg ggaagcagac 360
   ccgaccaatc ctgccttaag agaggagatg cgcattcaat tcaatgacat gaacagcgcg 420
   ctgacgaccg caattccgct cttcgccgtt cagaattacc aagttcctct tttatccgtg 480
   tacgtgcagg ctgccaacct gcacttgtcg gtgctccgcg atgtctccgt gttcggacaa 540
   cggtggggct ttgatgccgc aactatcaat agtcgttata atgatctgac taggcttatt 600
   ggcaactata ccgattatgc tgttcgctgg tacaacacgg gtctcgaacg tgtctgggga 660
   ccggattcta gagattgggt caggtacaac cagttcaggc gagagttgac actaactgtc 720
   ctagacattg tcgctctctt tcccaactac gactctaggc gctacccaat ccgtactgtg 780
   tcacaattga cccgggaaat ctacacaaac ccagtcctcg agaacttcga cggtagcttt 840
   cgaggctcgg ctcagggcat agagagaagc atcaggtctc cacacctgat ggacatattg 900
   aacagtatca cgatctacac cgatgcgcac cgcggttatt actactggtc agggcatcag 960
   atcatggcat cacccgttgg gttctctgga ccagaattca ctttcccact ttacgggact 1020
   atgggcaatg cagctccaca acaacgtatt gttgctcaac tcggtcaggg cgtgtataga 1080
   accttgtcca gcactctata taggagacct ttcaacatcg gcatcaacaa tcaacaattg 1140
   tctgtgcttg acgggacaga atttgcctat ggaacctcct caaatctgcc atccgctgtc 1200
   tacagaaaga gcggaacagt tgatagcttg gatgagatcc ctccacagaa caacaacgtt 1260
   ccacctaggc aagggtttag ccatcgcctt agccatgtgt ccatgttccg ttcaggcttt 1320
   agtaatagca gcgttagtat catcagagct ccgatgttct cttggataca tcgtagtgct 1380
   gagtttaaca acataattgc atccgatagc attactcaga tcccagctgt caaggggaac 1440
   tttctcttta atggttctgt catttcagga ccaggattca ctggaggcga cttggttagg 1500
   ctgaattctt ccggcaacaa catccagaat agagggtata ttgaagtgcc cattcacttc 1560
   ccatcgacat ctaccagata tcgtgttcgt gtaaggtatg cctctgttac ccctattcac 1620
   ctcaacgtca attggggtaa ttcctccatc ttttccaata cagtaccagc gacagctaca 1680
   tccttggata atctccaatc tagcgatttc ggttacttcg aaagtgccaa tgccttcacc 1740
   tcttccctag gtaacatagt aggtgttaga aatttctccg gaaccgccgg agtgataatc 1800
   gaccgcttcg aattcattcc cgttactgca acgctcgagg cagagtctga cttggaaaga 1860
   gcacagaagg cggtgaatgc tctgttcact tcgtccaatc agattgggct caagacagat 1920
   gtgactgact atcacatcga tcgcgtttcc aaccttgttg agtgcctctc tgatgagttc 1980
   tgtttggatg agaagaagga gttgtccgag aaggtcaaac atgctaagcg acttagtgat 2040
   gagcggaact tgcttcaaga tcccaacttt cgcgggatca acaggcaact agatcgtgga 2100
   tggaggggaa gtacggacat caccattcaa ggaggtgatg atgtgttcaa ggagaactat 2160
   gttacgctct tgggtacctt tgatgagtgc tatccaacat acctgtacca gaagatagat 2220
   gaatcgaaac tcaaagccta cacaagatac cagttgagag gttacatcga ggacagtcaa 2280
   gaccttgaga tctacctcat cagatacaac gccaaacatg agacagtcaa tgtgcctggg 2340
   acgggttcac tctggccact ttcagcccca agtcccatcg gcaagtgtgc ccatcactca 2400
   caccacttct ccttggacat agacgttggc tgtaccgacc tgaacgaaga cctcggtgtg 2460
   tgggtgatct tcaagatcaa gactcaagat ggccatgcca ggctaggcaa tctggagttt 2520
   ctagaagaga aaccacttgt tggagaagcc ctcgctagag tgaagagggc tgagaagaag 2580
   tggagggaca agagagagaa gttggaatgg gaaacaaaca ttgtgtacaa agaagccaaa 2640
   gaaagcgttg acgctctgtt tgtgaactct cagtatgata ggctccaagc tgataccaac 2700
   atagctatga ttcatgctgc agacaaacgc gttcatagca ttcgggaagc ttaccttcct 2760
   gaacttagcg tgattccggg tgtcaatgct gctatctttg aagagttaga agggcgcatc 2820
   ttcactgcat tctccttgta tgatgcgagg aatgtcatca agaatggtga cttcaacaat 2880
   ggcctatcct gctggaatgt gaaagggcac gtagatgtag aagaacagaa caatcaccgc 2940
   tctgtccttg ttgttcctga gtgggaagca gaagtttcac aagaagttcg tgtctgtcct 3000
   ggtcgtggct acattcttcg tgttaccgcg tacaaagaag gatacggaga aggttgcgtc 3060
   accatacacg agattgagaa caacaccgac gagctgaagt tcagcaactg cgtcgaggag 3120
   gaagtctacc caaacaacac cgtaacttgc aatgactaca ctgcgactca agaggagtat 3180
   gagggtactt acacttctcg caatcgagga tacgatggag cctatgagag caactcttct 3240
   gtacccgctg actatgcatc agcctatgag gagaaggctt acaccgatgg acgtagggac 3300
   aatccttgcg aatctaacag aggctatggg gactacacac cgttaccagc cggctatgtc 3360
   accaaagagt tagagtactt tccagaaacc gacaaggttt ggattgagat tggagaaacg 3420
   gaaggaacat tcattgttga tagcgtggag ttacttctga tggaggaa 3468